Europäisches Patentamt

European Patent Office

Office européen des brevets

(19)

(11) Veröffentlichungsnummer: **0 078 451**
**B1**

(12) # EUROPÄISCHE PATENTSCHRIFT

(45) Veröffentlichungstag der Patentschrift:
**17.04.85**

(51) Int. Cl.⁴: **G 01 N 33/86, C 12 Q 1/56**

(21) Anmeldenummer: **82109676.5**

(22) Anmeldetag: **20.10.82**

(54) **Reagenz für die Bestimmung des Ristocetin-Cofaktors (v. Willibrand-Faktor).**

(30) Priorität: **22.10.81 DE 3141894**

(43) Veröffentlichungstag der Anmeldung:
**11.05.83 Patentblatt 83/19**

(45) Bekanntmachung des Hinweises auf die Patenterteilung:
**17.04.85 Patentblatt 85/16**

(84) Benannte Vertragsstaaten:
**AT BE CH DE FR GB IT LI LU NL SE**

(56) Entgegenhaltungen:
**DE - A - 2 546 166**
**US - A - 4 145 185**

**CLINICAL CHEMISTRY, Vol. 25, No. 11, Nov. 1979, Easton, Pennsylvania, USA**

(73) Patentinhaber: **BEHRINGWERKE Aktiengesellschaft, Postfach 1140, D-3550 Marburg 1 (DE)**

(72) Erfinder: **Fuhge, Peter, Dr., Mühlenstrasse 10, D-3551 Lahntal (DE)**
Erfinder: **Braun, Konrad, Im Steu 8, D-3557 Ebsdorfergrund (DE)**
Erfinder: **Becker, Udo, Dr., Schmaedelstrasse 17, D-8000 München 60 (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

## Beschreibung

Die Erfindung betrifft ein Mittel zur Bestimmung des Ristocetin-Cofaktors, auch als v. Willebrand-Faktor oder F VIII R:RCF bezeichnet. Dieses enthält gegerbte, mit einem Proteasen-Inhibitor und gegebenenfalls einem Farbstoff behandelte Thrombozyten und Ristocetin A.

Für die Diagnose des v. Willebrand-Syndroms (WS) ist die Bestimmung der Ristocetin-Cofaktor (F VIII R:RCF)-Aktivität geeignet. Bekannte Verfahren beruhen darauf, daß das Antibiotikum Ristocetin A in humanem plättchenreichen Plasma die Aggregation von Plättchen induziert, nicht aber in Plasma von Menschen mit v. Willebrand-Syndrom.

Der Ristocetin-Cofaktor kann entsprechend dem Stand der Technik wie folgt bestimmt werden:

Humanes Citratplasma wird einer Suspension gewaschener humaner Plättchen zugesetzt, die Aggregation der Plättchen durch Zugabe von Ristocetin ausgelöst und über die Transmissionsänderung in einem Photometer mit einer Einrichtung zum Messen von Aggregationen (Aggregometer) gemessen. Die Transmission nimmt durch die Aggregation der Plättchen zu. Um eine Bezugskurve zu erhalten, werden abgestufte, gepoolte Human-Plasmaverdünnungen verwendet. Die Aggregation kann als Zunahme der Transmission in der Zeiteinheit gemessen und gegen die Plasmaverdünnung aufgetragen werden. Von der Bezugskurve kann der Gehalt eines Patentenplasmas an Ristocetin-Cofaktor abgelesen werden.

Nachteile solcher Verfahren sind, daß sie erfahrenes technisches Personal, exaktes Arbeiten und einen Schreiber erfordern, der die Transmissionsänderung bei der Aggregation genau wiedergibt, da ein Fehler in der Steigungsmessung von 1 Grad einem Fehler von 10% in der Ristocetin-Cofaktor-Konzentration entsprechen kann.

Es ist außerdem sehr aufwendig, die für das Verfahren erforderlichen frischen Blutplättchensuspensionen mit konstanter Plättchenzahl bereitzustellen. Diese Suspensionen sind nur begrenzt lagerfähig.

Ein weiterer Nachteil des genannten Verfahrens ist das große Testvolumen, das große Mengen des teuren Ristocetins erfordert. Da die Ristocetinkonzentration nur in einem engen Bereich variiert werden kann, ist eine Einsparung nur durch eine Verminderung des Testvolumens möglich. Ein Agglutinationstest auf Testplatten, in der Art eines Latex-Testes, verringert das Testvolumen auf ein Zehntel, ohne die Empfindlichkeit des Testes zu beeinflussen. Frische Plättchensuspensionen sind hierfür nicht geeignet, da sie bereits Plättchenaggregate enthalten, die eine Agglutination der Nullkontrolle hervorrufen.

Die Bestimmung des Ristocetin-Cofaktors wurde bereits durch den Einsatz gegerbter Plättchen weiter vereinfacht. Durch Fixierung mit Glutardialdehyd (DE-OS 2 546 166) oder Formalin (Allain et al., J. Lab. Clin. Med. (1975), 85, 318) erhält man Blutplättchensuspensionen, die auch nach Lagerung aktiv sind, insbesondere wenn man sie lyophilisiert. Wenn man solchermaßen lyophilisierte Plättchen resuspendiert, findet man allerdings auch vermehrt Plättchenaggregate, die die Verwendung in einem Agglutinationstest beeinträchtigen, da auch hier die Nullkontrolle spontane Agglutinationen zeigt. Auch wenn Plättchen zusammen mit Ristocetin lyophilisiert werden, bilden sich bei der Resuspendierung spontan Aggregate, die ihren Einsatz in einem Agglutinationstest unmöglich machen.

Es wurde nun gefunden, daß die beschriebenen Nachteile der bekannten Verfahren durch die Verwendung eines Reagenzes vermieden werden, das auf dem überraschenden Befund beruht, daß sich mit einem Proteaseninhibitor und einem für die Fixierung von Proteinen bekannten Gerbstoff behandelte Thrombozyten mit Ristocetin und Hilfsmitteln lyophilisieren und längere Zeit bei Raumtemperatur oder sogar bei 37° C lagern lassen, ohne ihre Brauchbarkeit zur Bestimmung des Ristocetin-Cofaktors einzubüßen.

Gegenstand der Erfindung ist ein Mittel zur Bestimmung des Ristocetin-Cofaktors (v. Willebrand-Faktor; F VIII R:RCF), dadurch gekennzeichnet, daß es mit einem Proteaseninhibitor und einem für die Fixierung von Proteinen bekannten Gerbstoff, und gegebenenfalls einem für die Anfärbung von Proteinen bekannten Farbstoff, behandelte Thrombozyten und Ristocetin A enthält.

Das erfindungsgemäße Reagenz ermöglicht eine semiquantitative Bestimmung des Ristocetin-Cofaktors nach der Agglutinationsmethode und mit nur zwei Pipettierschritten (Reagenz und Probe) und unter Verwendung von nur wenig Ristocetin.

Es hat sich als günstig erwiesen, die Plättchen mit einer Lösung eines $C_1$—$C_4$-Alkanaldehyds, vorzugsweise Formaldehyd, oder eines $C_2$—$C_6$-Dialdehyds, vorzugsweise Glutardialdehyd, in einer Mindestkonzentration von 0,2% (w : v) mindestens zwei Stunden lang zwischen 4° C und 37° C zu fixieren und außerdem mit einem Proteasen-Inhibitor, vorzugsweise Diisopropylfluorophosphat, in einer Mindestkonzentration von $10^{-6}$ M zu behandeln. Die Thrombozyten werden gegebenenfalls mit einem für die Anfärbung von Proteinen bekannten Farbstoff, besonders mit einem in der Gelelektrophorese verwendeten Farbstoff wie Coomassie Blau oder einem Reaktivfarbstoff wie Remazoltürkisblau® EEAD 505 gefärbt. Dabei ist die Farbstoffkonzentration mindestens 0,05 mg/ml und die Färbedauer ist höchstens 24 Stunden.

Der Suspension, die $10^7$ bis $10^{11}$ Thrombozyten/ml enthält, wird so viel Ristocetin A zugesetzt, daß sie 0,2—10 mg/ml, vorzugsweise 2,5 mg/ml, enthält. Die Suspension wird zusammen mit Lyophilisationshilfen getrocknet. Als Hilfsmittel werden pro ml Suspension 1 bis 100 mg einer oder mehrerer Aminosäuren, vorzugsweise Glycin und Natrium-Glutaminat, 2 bis 30 mg eines Zuckers, vorzugsweise Saccharose, und 0,5 bis 10 mg eines Schutzkolloids, vorzugsweise Humanalbumin, zugesetzt.

Das folgende Beispiel soll die Erfindung näher erläutern:

1,3 l Thrombozytenkonzentrat werden in 4 l Phosphatpuffer 0,15 M, pH 7,2, suspendiert, und die Erythrozyten werden abzentrifugiert. Die Waschung und Zentrifugation wird zweimal wiederholt und anschließend der Überstand auf $3 \times 10^9$ Plättchen/ml eingestellt. Unter strengen Vorsichtsmaßnahmen (Abzug, Handschuhe) wird Diisopropylfluorophosphat auf eine Endkonzentration von $10^{-3}$ M zugegeben und 1 Stunde bei Raumtemperatur gerührt. Nach Zentrifugation wird der Überstand verworfen und der Niederschlag zweimal mit Phosphatpuffer gewaschen. Die Plättchensuspension wird mit einer 35%igen Formaldehydlösung auf eine Konzentration von 4% Formaldehyd eingestellt und 44 Stunden bei 4°C leicht gerührt. Anschließend wird ausgiebig gegen Phosphatpuffer dialysiert. Die Plättchenkonzentration wird auf $3 \times 10^9$/ml eingestellt und zu 1 l Suspension 2,5 g Ristocetin A, 10 g Saccharose, 10 g Glycin, 16,7 g N-Glutaminat und 2 g Humanalbumin zugefügt. Nach Einfrieren wird lyophilisiert. Die Plättchenzahl vor und nach Lyophilisation bleibt konstant.

Die Plättchen können nach Fixation auch gefärbt werden, beispielsweise mit Remazolblau® (Hoechst EE AD 505), das die Plättchen intensiv blau färbt und die Empfindlichkeit des Testes erhöht.

Die Plättchen können durch Zugabe von 0,25 mg/ml Remazolblau zu der fixierten und dialysierten Plättchensuspension und 30minütiges Rühren gefärbt werden. Danach wird zentrifugiert und der Niederschlag gewaschen. Nach Zentrifugation werden die Plättchen wie die ungefärbten Plättchen weiterbehandelt.

Auch bei diesem Reagenz sind nach Rekonstitution in $H_2O$ keine Aggregate zu beobachten.

Bestimmung des Ristocetin-Cofaktors

Dazu wird das Lyophilisat auf 1 ml Ausgangssuspension mit 1 ml Wasser rekonstituiert, einer geometrischen Verdünnungsreihe von gepooltem Humanplasma zugesetzt und die Agglutination ausgewertet.

Testansatz:
50 μl Plasmaverdünnung und 50 μl Reagenz werden auf einer Glasplatte gut gemischt, 1 Minute geschüttelt und nach einer Minute Ruhe die Titerstufe abgelesen.

Stabilität nach Belastungsversuch:
Nach 4 Wochen Lagerung bei 37°C werden die gleichen Titerstufen gefunden.

## Patentansprüche

1. Mittel zur Bestimmung des Ristocetin-Cofaktors, dadurch gekennzeichnet, daß es mit einem Serin-Proteinasen-Inhibitor und einem für die Fixierung von Proteinen bekannten Gerbstoff, und gegebenenfalls einem für die Anfärbung von Proteinen bekannten Farbstoff, behandelte Thrombozyten und Ristocetin A enthält.

2. Mittel nach Anspruch 1, dadurch gekennzeichnet, daß der Proteinasen-Inhibitor Diisopropylfluorophosphat ist.

3. Mittel nach Anspruch 1, dadurch gekennzeichnet, daß es lyophilisiert ist.

4. Mittel nach Anspruch 1, dadurch gekennzeichnet, daß der Farbstoff für die Thrombozyten Remazoltürkisblau® EEAD 505 ist.

5. Mittel nach Anspruch 3, dadurch gekennzeichnet, daß es 1 bis 100 mg Aminosäuren, 2 bis 30 mg eines Zuckers und 0,5 bis 10 mg eines Schutzkolloids auf etwa $3 \times 10^9$ Thrombozyten enthält.

## Claims

1. An agent for determining ristocetin cofactor, which contains platelets which have been treated with a serine proteinase inhibitor and with a tanning agent known for fixing proteins and, optionally, with a dyestuff known for staining proteins, and ristocetin A.

2. The agent as claimed in claim 1, wherein the proteinase inhibitor is diisopropyl fluorophosphate.

3. The agent as claimed in claim 1, which has been freeze dried.

4. The agent as claimed in claim 1, wherein the dyestuff for the platelets is Remazol® turquoise blue EEAD 505.

5. The agent as claimed in claim 3, wherein it contains 1 to 100 mg of aminoacids, 2 to 30 mg of a sugar and 0.5 to 10 mg of a protective colloid to about $3 \times 10^9$ platelets.

## Revendications

1. Agent pour la détermination du cofacteur de ristocétine, caractérisé en ce qu'il contient des thrombocytes traités avec un inhibiteur de protéinases sériques et un tannin connu pour la fixation des protéines, et éventuellement avec un colorant connu pour la coloration des protéines, et de la ristocétine A.

2. Agent selon la revendication 1, caractérisé en ce que l'inhibiteur des protinases est le fluorophosphate de diisopropyle.

3. Agent selon le revendication 1, caractérisé en ce qu'il est lyophilisé.

4. Agent selon la revendication 1, caractérisé en ce que le colorant pour les thrombocytes est le bleu turquoise Remazol® EEAD 505.

5. Agent selon la revendication 3, caractérisé en ce qu'il contient de 1 à 100 mg d'acides aminés, de 2 à 30 mg d'un sucre et de 0,5 à 10 mg d'un colloïde protecteur pour environ $3 \times 10^9$ thrombocytes.